# EUROPEAN PATENT SPECIFICATION

(11) **EP 3 116 627 B1**
(45) Date of publication and mention of the grant of the patent: **26.12.2018**
(21) Application number: 15718587.7
(22) Date of filing: 10.03.2015
(51) Int. Cl.: B01D 53/14, B01D 53/58, B01D 53/96, C07C 273/04

(54) **RECOVERY METHOD FOR PROCESSING A GAS STREAM FROM A UREA PLANT SOLIDIFICATION UNIT**
RÜCKGEWINNUNGSVERFAHREN ZUR VERARBEITUNG EINES GASSTROMS AUS EINER SOLIDIFIZIERUNGSEINHEIT EINER HARNSTOFFANLAGE
PROCÉDÉ DE RÉCUPÉRATION PERMETTANT LE TRAITEMENT D'UN COURANT DE GAZ PROVENANT D'UNE UNITÉ DE SOLIDIFICATION D'INSTALLATION DE PRODUCTION D'URÉE

(30) Priority: 10.03.2014 IT MI20140369
(43) Date of publication of application: 18.01.2017
(73) Proprietor: SAIPEM S.p.A., 20097 San Donato Milanese (MI) (IT)
(72) Inventor: BRUNO, Lorenzo, I-20097 San Donato Milanese (IT); CARLESSI, Lino, I-24044 Dalmine (IT)
(74) Representative: Cernuzzi, Daniele
(86) International application number: PCT/IB2015/051739
(87) International publication number: WO 2015/136444

(56) References cited:
- EP-A1- 2 386 346
- WO-A1-2011/012324
- DE-A1-102007 022 462
- US-A1- 2011 091 369
- US-A1- 2012 039 787

## Description

### TECHNICAL FIELD

The present invention relates to a recovery method for processing a gas stream from a solidification unit of a urea plant, recovering from said gas stream components to reuse in the same recovery method and/or in the urea plant.

### BACKGROUND ART

As is known, urea is produced on an industrial scale via processes based on the reaction, under high-temperature and high-pressure conditions, between carbon dioxide and ammonia to form ammonium carbamate, and the subsequent decomposition reaction of the ammonium carbamate to provide urea and water. In a typical urea production plant (urea plant), these processes are generally carried out in a urea synthesis reactor; the aqueous urea solution produced in the synthesis reactor is then progressively concentrated, with recover of unconverted reagents, in one or more recovery sections, for example in a high-pressure section, a medium-pressure section and a low-pressure section; finally, the urea is solidified in a solidification section, which normally includes a granulator or a prilling tower.

In particular, the urea leaving the synthesis reactor is subsequently concentrated to values approximately above 95% by weight (typically 96-99. 7%) before being sent to the solidification section (granulator or prilling tower) to obtain the final commercial product, in the form of urea granules or prills.

Normally, the urea sent to the granulator or prilling tower is in a liquid state and solidifies by means of a hot stream of ambient air.

When the air used for solidification leaves the solidification section, it contains urea powder and ammonia released by the liquid urea during the cooling and solidification process.

Before releasing this air into the atmosphere, it is therefore opportune to purify it by removing the urea powder and ammonia it contains.

After having removed the urea powder, usually in a first scrubber where an aqueous solution of liquid urea circulates, it is known to remove the ammonia in a second scrubber, where a solution containing ammonia salts circulates.

For example, it is known to circulate a solution containing ammonium sulphate and ammonium bisulphate in the ammonia removal scrubber; the ammonia in the air, reacting with the ammonium bisulphate, forms ammonium sulphate and the purified air may be released into the atmosphere, while the solution of ammonium sulphate is recirculated in the scrubber to continue the treatment and, in part, removed as a by-product. In order to maintain the correct ratio between ammonium sulphate and ammonium bisulphate in the recirculating solution, it is necessary to add sulphuric acid, keeping the pH of the solution in the range 0 to 7. The by-product obtained is a solution of ammonium sulphate. The problem of using the solution of ammonium sulphate obtained arises; in fact, ammonium sulphate is a reaction by-product of little commercial interest, hygroscopic if water is removed and therefore difficult to process from the chemical-physical viewpoint. Furthermore, sulphuric acid, with the associated costs of provisioning and management, is consumed to top up the ammonium bisulphate.

It is also known to use solutions containing ammonium phosphates in the scrubber, in particular feeding a solution containing phosphoric acid and monoammonium phosphate at the top of the scrubber, and a solution containing monoammonium phosphate and diammonium phosphate at the bottom of the scrubber. The ammonia in the air, reacting with the ammonium monosphosphate and phosphoric acid, and forms diammonium phosphate and monoammonium phosphate, respectively; the purified air may be released into the atmosphere, while the solution of diammonium phosphate and monoammonium phosphate is recirculated in the scrubber to continue the treatment and, in part, removed as a by-product. In order to maintain the correct ratio between phosphoric acid and monoammonium phosphate in the solution at the top of scrubber and an adequately low pH in the recirculating solution, phosphoric acid is added, with the associated costs of provisioning and management. The by-product obtained is a solution of diammonium phosphate and monoammonium phosphate, this also of little commercial interest.

For example, WO 2011/012324 A1, US 2012/039787 A1 and US 2011/091369 A1 disclose methods for the recovery of ammonia contained in a gaseous stream produced in a synthesis process of urea. In all cases, the gas stream is washed in a scrubber; then, the aqueous solution exiting from the scrubber and containing ammonium salts is treated to obtain a regenerated solution which is recycled to the scrubber. In all cases, in order to maintain an appropriate pH in the scrubber, it is necessary to add to the recirculating solution some components.

In the methods mentioned herein, as with other similar ones, it is therefore necessary to replenish reactants (with consequent provisioning and management costs) and the problem still arises of using the by-products, of little commercial interest.

### DISCLOSURE OF INVENTION

One object of the present invention is to provide a recovery method for processing a gas stream from a solidification unit of a urea plant, in particular for removing ammonia from said gas stream, without the described drawbacks of the known art.

In particular, an object of the invention is to provide a recovery method that enables efficiently recovering and reusing each component used or produced in the recovery method and/or in the urea plant.

The present invention therefore relates to a recovery method for processing a gas stream from a solidification unit of a urea plant as defined in appended claim 1.

Additional preferred features of the invention are indicated in the dependent claims.

The method of the invention allows avoiding the formation of any by-product, as each component is recovered and reused; furthermore, there is not even a requirement to add reactants from the outside, because the method only uses components already present or produced.

### BRIEF DESCRIPTION OF THE DRAWINGS

Further characteristics and advantages of the present invention will become clear from the description of the following non-limitative embodiments, with reference to the figures in the accompanying drawings, in which:
- Figure 1 is a schematic view of a first embodiment of a recovery system which can be used for processing a gas stream from a solidification unit of a urea plant in accordance with the method of the invention;
- Figure 2 is a schematic view of a second embodiment of a recovery system which can be used for carrying out the method in accordance with the invention;

- Figure 3 is a schematic view of a third embodiment of a recovery system which can be used for carrying out the method in accordance with the invention;
- Figure 4 is a schematic view of a fourth embodiment of a recovery system which can be used for carrying out the method in accordance with the invention;
- Figure 5 is a schematic view of a fifth embodiment of a recovery system which can be used for carrying out the method in accordance with the invention;
- Figure 6 is a schematic view of a sixth embodiment of a recovery system which can be used for carrying out the method in accordance with the invention;
- Figure 7 is a schematic view of a seventh embodiment of a recovery system which can be used for carrying out the method in accordance with the invention.

### BEST MODE FOR CARRYING OUT THE INVENTION

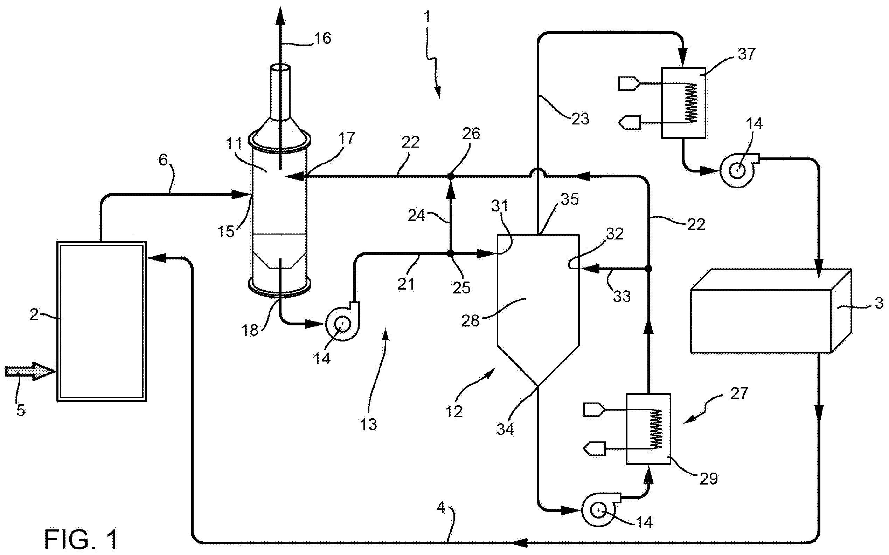

Figure 1 schematically shows a recovery system 1 for processing a gas stream leaving a solidification unit 2 of a urea plant 3 (i.e. a urea production plant) and recovering components from said gas stream to reuse in the recovery system 1 and in the urea plant 3.

The urea plant 3 is, in itself, essentially known and shall therefore be neither described nor illustrated in detail. In general terms, the urea plant 3 comprises: a urea synthesis reactor where the reaction of synthesis of urea from ammonia and carbon dioxide takes place; recovery sections (for example, in particular a high-pressure section, a medium-pressure section and a low-pressure section), in which the urea solution produced in the synthesis reactor is progressively concentrated, with the removal of water and unreacted ammonia and carbon dioxide, and recirculation of the recovered components; and a vacuum section equipped with a vacuum system and connected to a waste-water treatment section.

The urea plant 3 is connected via a urea feed line 4 to the solidification unit 2, which includes, for example, a prilling tower (as schematically shown in Figure 1) or a granulator, to which the molten urea produced in the urea plant 3 is sent to obtain solid urea in granules or prills.

The solidification unit 2 is fed with a stream of cooling air, schematically represented by the arrow 5 in Figure 1, which enters the solidification unit 2 through suitable air inlet openings, for the purpose of solidifying the molten urea.

From the solidification unit 2 a gas stream exits that is basically formed by the cooling air used in the solidification unit 2 and contains urea powder and ammonia (released by the urea during the cooling and solidification process).

After removal of the urea powder (in a suitable unit, for example a scrubber, known and in itself not relevant to the present invention and therefore neither shown nor described for simplicity), the gas stream leaving the solidification unit 2 (intended, here and hereinafter, as the gas stream leaving the solidification unit 2 and purified of urea powder), is sent to the recovery system 1 through a gas line 6 that operationally connects the solidification unit 2 to the recovery system 1.

The recovery system 1 comprises a scrubber 11 connected to the solidification unit 2 to process the gas stream from the solidification unit 2, a heat treating unit 12 operating on a recovery solution extracted by the scrubber 11, and a circuit 13, equipped with circulation pumps 14, which connects the scrubber 11, the heat treating unit 12 and the urea plant 3.

The scrubber 11 has a gas inlet 15 connected to the gas line 6, a gas outlet 16 (connected to a chimney for example), a reactant inlet 17 and a bottom outlet 18.

The function of the scrubber 11 is to remove ammonia from the gas stream leaving the solidification unit 2; the removal of ammonia in the scrubber 11 takes place through a reaction with at least one reactant capable of capturing the ammonium group, as will be described in greater detail hereinafter.

The heat treating unit 12 is connected in a loop with the scrubber 11 via an inlet line 21 and a return line 22. In particular, the heat treating unit 12 is connected to the bottom outlet 18 of the scrubber 11 by the inlet line 21, to receive the recovery solution produced in the scrubber 11 and subject it to a heat treatment in a controlled temperature range, and to the reactant inlet 17 of the scrubber 11 by the return line 22, to return a reactant composition, produced in the heat treating unit 12 and containing at least one ammonia salt, to the scrubber 11.

The heat treating unit 12 is also connected to the urea plant 3 by an ammonia line 23.

The circuit 13 thus comprises: the inlet line 21 and the return line 22, which connect the heat treating unit 12 and the scrubber 11; and the ammonia line 23, which connects the heat treating unit 12 to the urea plant 3.

In addition, the circuit 13 comprises a bypass line 24 that connects the inlet line 21 to the return line 22, excluding the heat treating unit 12 (i.e. it connects a branch point 25 of the inlet line 21 located upstream of the heat treating unit 12 to an inflow point 26 of the return line 22 located downstream of the heat treating unit 12).

The heat treating unit 12 is equipped with heating means 27 to heat the recovery solution up to a controlled temperature, or within a controlled temperature range, such as to separate the ammonia, to be reused in the urea plant 3, and produce a molten reactant composition, containing at least one ammonia salt, to be recirculated to the scrubber 11 and used to remove ammonia from the gas stream coming from the solidification unit 2.

In the embodiment in Figure 1, the heating means 27 of the heat treating unit 12 comprise an oven 28, constituted, for example (but not necessarily), by a crucible, possibly lined with a refractory material, and a heat exchanger 29, in particular an indirect-contact heat exchanger in which a heating fluid circulates, this fluid being constituted, for example, by diathermic oil, steam (for example, medium-pressure steam taken from the medium-pressure section of the urea plant 3), combustion fumes, or some other fluid.

The oven 28 has a main inlet 31 connected to the inlet line 21 and therefore to the bottom outlet 18 of the scrubber 11, a secondary inlet 32 connected to the heat exchanger 29 via a service line 33, a bottom outlet 34 connected to the return line 22, and a top outlet 35 connected to the ammonia line 23.

In particular, the heat exchanger 29 is located along the return line 22 and is connected in a loop with the oven 28 via service line 33, which branches from the return line 22 downstream of the heat exchanger 29 and connects the heat exchanger 29 to the secondary inlet 32 of the oven 28.

The ammonia line 23 is equipped with a condensation unit 37, for example one fed with cold water, which also acts as a vacuum system for extracting gas streams from the oven 28 (clearly, a different vacuum system or a pressurized extraction system could also be provided for extraction).

Circulation pumps 14 are positioned, for example, on the inlet line 21, the return line 22 (in particular, between the oven 28 and the heat exchanger 29) and the ammonia line 23.

In use, the solidification unit 2 receives molten urea from the urea plant 3 through the urea feed line 4 and is fed with cooling air 5. From the solidification unit 2 solid urea (in granules or prills) is collected and a gas stream exits that is basically formed by the cooling air used in the solidification unit 2 and containing ammonia, which is sent to the recovery system 1 (more precisely, to the scrubber 11) through the gas line 6.

The gas stream leaving the solidification unit 2 (after being preliminarily treated for the removal of urea powder), is processed in the scrubber 11 to remove the ammonia it contains via a reaction with at least one reactant, in particular an acid or an acid salt (a salt that contains hydrogen residues of the acid it derives from), capable of capturing the ammonium group, i.e. capable of reacting with the ammonia to form an ammonia salt. In this way, the gas stream produced in the solidification unit 2 is freed from ammonia inside the scrubber 11 and may be discharged into the atmosphere (through the gas outlet 16), and an aqueous solution of one or more ammonia salts is formed, which constitutes the recovery solution that is recovered in the heat treating unit 12.

In the heat treating unit 12, the recovery solution is heated to a controlled temperature to separate the ammonia, to be reused in the urea plant 3, and produce a molten reactant composition containing at least one ammonia salt, to recirculate to the scrubber 11 and use for removing ammonia from the gas stream coming from the solidification unit 2.

The temperature at which the heat treating unit 12 operates is such as to cause degradation of the ammonia compounds contained in the recovery solution to give gaseous ammonia, but to avoid degradation of the ammonia salts (or the corresponding acids) to give gaseous compounds that would pollute the recovered ammonia.

For example, the scrubber 11 produces an aqueous solution of ammonium sulphate (AS) with an approximate ammonium sulphate concentration of around 40%; this solution constitutes the recovery solution leaving the scrubber 11 and which is taken from the bottom outlet 18 of the scrubber 11 and sent through the inlet line 21 to the heat treating unit 12.

The heat treating unit 12 serves to obtain ammonia (in the gaseous phase, i.e. ammonia vapours) from the recovery solution containing ammonium sulphate (AS) and coming from the scrubber 11, the ammonia then being sent through the ammonia line 23 to the urea plant 3 for the production of urea, and a reactant composition, containing ammonium bisulphate (ABS) and (in a smaller amount) ammonium sulphate (AS), which is recirculated to the scrubber 11 through the return line 22 and reused in the scrubber 11 for removing ammonia from the gas stream coming from the solidification unit 2.

The ammonia and ammonium bisulphate are produced from ammonium sulphate according to the reaction:

(I) (NH₄)₂SO₄ + heat → (NH₄)HSO₄ + NH₃

This reaction starts to occur at temperatures higher than about 250°C. However, for temperatures higher than 350°C, undesired decomposition reactions start to occur, such as the following reactions, for example:

(II) (NH₄)₂SO₄ + heat → 2H₂O + 2NH₃ + SO₃

(III) 2 (NH₄)₂SO₄ + heat → 4H₂O + 4NH₃ + 2O₂ + 2SO₂

Since these reactions result in the formation of SO₃ or SO₂ mixed with the ammonia vapours, which complicates the subsequent recovery and use of the ammonia in the urea plant 3, it is preferable to facilitate the kinematics of reaction (I) and avoid reactions (II) and (III); to this end, the heat treating unit 12 operates within a temperature range of 300-320°C, in particular at a temperature of around 312°C.

The heat necessary for reaction (I) is provided in the heat treating unit 12.

A recycle portion of the recovery solution (ammonium sulphate solution) produced in the scrubber 11 is recirculated to the scrubber 11 through the bypass line 24; the remainder is sent to the heat treating unit 12 and, in particular, to the oven 28, where the ammonia is separated to obtain the reactant composition containing ammonium bisulphate (ABS) and ammonium sulphate (AS).

Therefore, with regard to the reactants necessary for removing the ammonia, the scrubber 11 is fed with the reactant composition produced in the heat treating unit 12, with the addition of a portion (recycle portion) of the recovery solution; the addition of fresh components is not required.

The gaseous ammonia produced in the heat treating unit 12 is extracted from the oven 28 through the top outlet 35, and, after running through the ammonia line 23, is condensed in the condensation unit 37 and finally sent to the urea plant 3, to be reused.

In embodiments not according to the invention, the recovery solution produced in the scrubber 11 is an aqueous solution of monoammonium phosphate (MAP) and diammonium phosphate (DAP), from which the heat treating unit 12 recovers ammonia (in the gaseous phase, i.e. ammonia vapours), which is sent to the urea plant 3, and a reactant composition containing monoammonium phosphate (MAP) and phosphoric acid (PA), which is reused in the scrubber 11 for the reaction to remove ammonia from the gas stream coming from the solidification unit 2.

In this case as well, the recovery solution (MAP-DAP solution) is taken from the bottom outlet 18 of the scrubber 11 and is sent through the inlet line 21 to the heat treating unit 12.

The heat treating unit 12 heats the recovery solution to a predetermined temperature, to cause degradation of the ammonia salts, with the consequent release of gaseous ammonia and the formation of a molten mixture containing MAP and phosphoric acid (PA).

The following reactions are exploited in the heat treating unit 12 to produce ammonia, MAP and phosphoric acid from the recovery solution:

(IV) (NH₄)₂HPO₄ + heat → (NH₄)H₂PO₄ + NH₃

(V) (NH₄)H₂PO₄ + heat → H₃PO₄ + NH₃

Even though these reactions start to take place at temperatures higher than about 125°C, in order to have a high degradation rate for the MAP-DAP solution, it is opportune to maintain a temperature above 158°C, the boiling temperature of phosphoric acid.

However, for temperatures higher than about 213°C, the formation of pyrophosphate, according to the following reaction, starts to become significant:

(VI) 2H₃PO₄ + heat → H₂O+ H₄P₂O₇

This reaction, together with any subsequent formation of metaphosphoric acid and phosphorus trioxide, results in a mixture with undesired chemical-physical characteristics (corrosion, high melting point, high viscosity, etc.), without even any significant advantages in reducing the pH of the recovery solution.

It is therefore opportune to keep the temperature below 213°C in order to avoid the significant formation of pyrophosphate and phosphorus trioxide.

In short, it is preferable to operate so as to favour the kinematics of reactions (IV) and (V) and avoid reaction (VI), by operating within a temperature range of 125-213°C and preferably 158-213°C.

Preferably, operations are performed at a maximum temperature of 190°C, the melting point of MAP, thereby always obtaining a molten MAP-PA mixture.

A recycle portion of the recovery solution (containing the molten MAP-DAP mixture) produced in the scrubber 11 is recirculated directly to the scrubber 11, through the bypass line 24, without passing through the heat treating unit 12 (in particular without passing through the oven 28); the remainder is sent to the heat treating unit 12 and, in particular, to the oven 28, where the ammonia is separated and the reactant composition containing the molten MAP-PA mixture is obtained. In particular, the dissociation of MAP and DAP, and the evaporation of the water entering with the recovery solution coming from the scrubber 11, take place in the oven 28.

The molten MAP-PA mixture produced in the oven 28 (except for the part that is used in the oven 28) is cooled and sent to the scrubber 11, in particular by mixing it with the recycle portion of the recovery solution leaving the scrubber 11. In this way, it is possible to keep the pH of the recovery solution below 5, suitable for efficient operation of the scrubber 11. If necessary, makeup water may be added to the reactant composition.

Thus, in this case as well, the addition of fresh reactants is not required (but only makeup water, if necessary).

Also in this case, the gaseous ammonia produced in the heat treating unit 12 is condensed in the condensation unit 37 and then sent to the urea plant 3, to be reused.

With the layout shown in Figure 1, given that the recovery solution coming from the scrubber 11 is an aqueous solution that is sent directly to the oven 28, it is also necessary to provide heat for the evaporation of the water present, which will then be found in the ammonia vapours leaving from the top outlet 35 of the oven 28. Clearly, the higher the concentration of the recovery solution, the lesser the heat needed to evaporate the water contained therein.

The recovery solution coming from the scrubber 11 may be brought to the operating temperature for degradation of the ammonia salt present (300-320°C for treating a recovery solution containing ammonium sulphate) and be kept molten by adjusting the temperature in various ways, other than that described above.

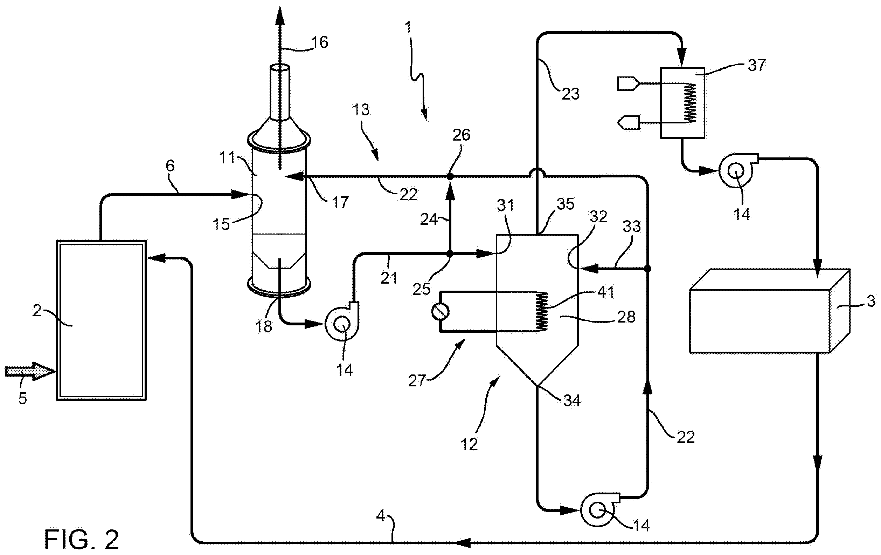

For example, in Figures 2-7, where details similar or identical to those already described are indicated with the same reference numerals, several embodiments are shown, in which the recovery system 1 has different configurations and includes various types of heat treating units 12.

All of the plant configurations shown may be utilized according to the method of the invention with the previously described reactant compositions containing ammonium sulphates (i.e. reactant compositions containing a solution of ABS and AS) .

In the embodiment in Figure 2, the heat treating unit 12 comprises an electric oven 28 with an electric resistance.

In particular, the oven 28 includes at least one electric resistance 41 placed inside the oven 28 and, for example, immersed in the molten bath that forms in the oven 28.

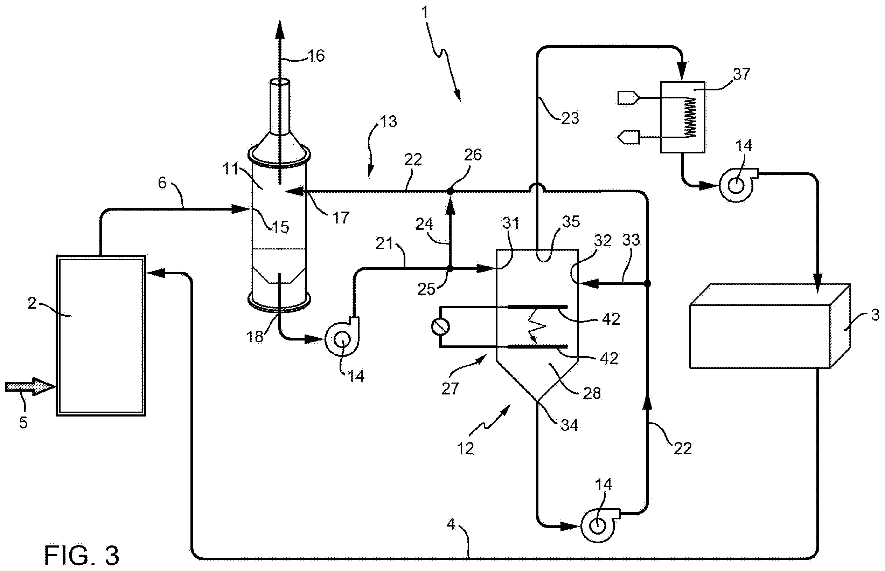

In the embodiment in Figure 3, the heat treating unit 12 comprises an electric oven 28 with immersed electrodes.

In particular, the oven 28 includes at least one pair of electrodes 42 (for example in graphite or equivalent materials), placed in oven 28 and immersed in the molten bath contained in the oven 28.

This embodiment is particularly cost-effective from the investment cost viewpoint and significantly reduces problems linked with materials corrosion.

In Figure 3, a pump 14 (which also provides for the recirculation of the reactant composition to the oven 28 and to the scrubber 11) is used for extraction of the reactant composition (solution of ABS and AS) from the oven 28.

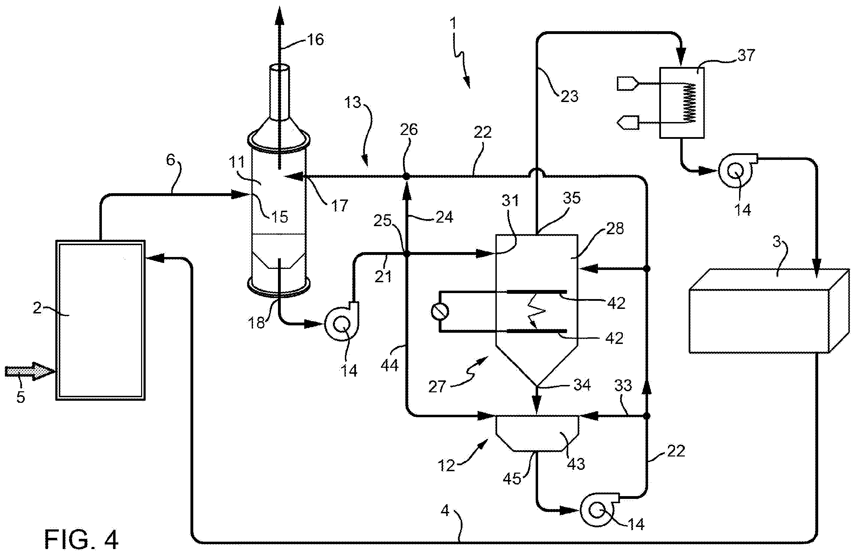

Also in the embodiment in Figure 4 the heat treating unit 12 comprises an electric oven 28 with immersed electrodes 42. The oven 28 is connected to a mixer 43 located, for example, underneath the bottom outlet 34 of the oven 28.

Similarly to that previously described, the oven 28 separates the ammonia and produces a molten mixture (a solution of ABS and AS) that in this case, however, before being recirculated to the scrubber 11, is mixed in the mixer 43 with a portion of the recovery solution coming from the scrubber 11, thereby incorporating an aqueous component (solution).

The molten mixture produced in the oven 28 is collected in the mixer 43, where, for example, it falls under gravity; an auxiliary line 44, which runs from the inlet line 21 upstream of the oven 28 (for example, from branch point 25), conveys a portion of the recovery solution leaving the scrubber 11 to the mixer 43.

The return line 22, equipped with a pump 14, connects an outlet 45 of the mixer 43 to the reactant inlet 17 of the scrubber 11.

The service line 33 runs from the return line 22 and recirculates a portion of the reactant composition circulating in the return line 22 (which in this case is, as indicated above, the aqueous solution formed in the mixer 43 and comprising the molten mixture produced in the oven 28) to the mixer 43. In this way, the pump 14 located on the return line 22 operates on the aqueous solution produced in the mixer 43 (and not on the molten mixture leaving the oven 28).

Also in this case, the bypass line 24, which runs from the inlet line 21 (for example from branch point 25) and merges into the return line 22 (in particular, at inflow point 26), recirculates a recycle portion of the recovery solution coming from the scrubber 11 directly back to the scrubber 11, without passing through the heat treating unit 12,.

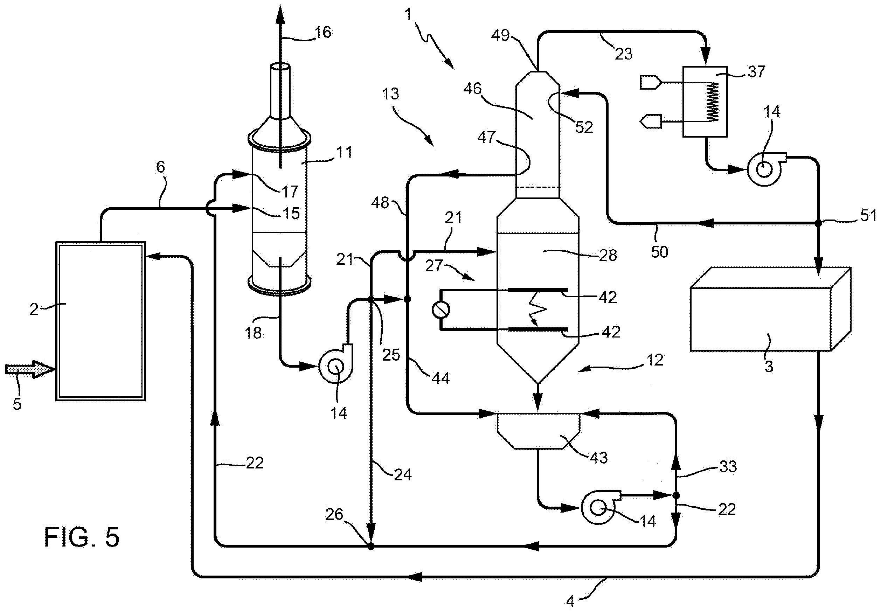

In the embodiment in Figure 5, the heat treating unit 12 still includes an electric oven 28 with immersed electrodes, associated with a mixer 43.

With respect to the previous embodiment in Figure 4, the heat treating unit 12 includes also a distillation column 46, placed, in particular, at the top of the oven 28 to separate the water from the ammonia in the gas stream leaving the oven 28, before the ammonia is condensed and sent to the urea plant 3.

The distillation column 46 receives the gaseous phase produced in the oven 28 (basically formed of ammonia and water) and separates the water, which flows out from a bottom outlet 47 of the distillation column 46 and, through a makeup line 48, flows into the auxiliary line 44 to feed the mixer 43, from the ammonia, which flows out from a top outlet 49 of the distillation column 46 and into the ammonia line 23.

A recirculation line 50 connects a branch point 51 of the ammonia line 23, located downstream of the condensation unit 37, to a top inlet 52 of the distillation column 46, to recirculate liquid ammonia to the distillation column 46.

The distillation column 46 does not need a bottom reboiler, as hot ammonia vapours coming from the oven 28 are used.

The mixer 43 is fed, via the auxiliary line 44 that runs from the inlet line 21 upstream of the oven 28 (for example, from branch point 25), with a portion of the recovery solution leaving the scrubber 11, with the addition of water extracted from the distillation column 46 and which flows into auxiliary line 44 through the makeup line 48.

As in the other embodiments, the bypass line 24, which connects the inlet line 21 to the return line 22 (connecting, in particular, branch point 25 to inflow point 26), recirculates a recycle portion of the recovery solution coming from the scrubber 11 directly back to the scrubber 11, without passing through the heat treating unit 12.

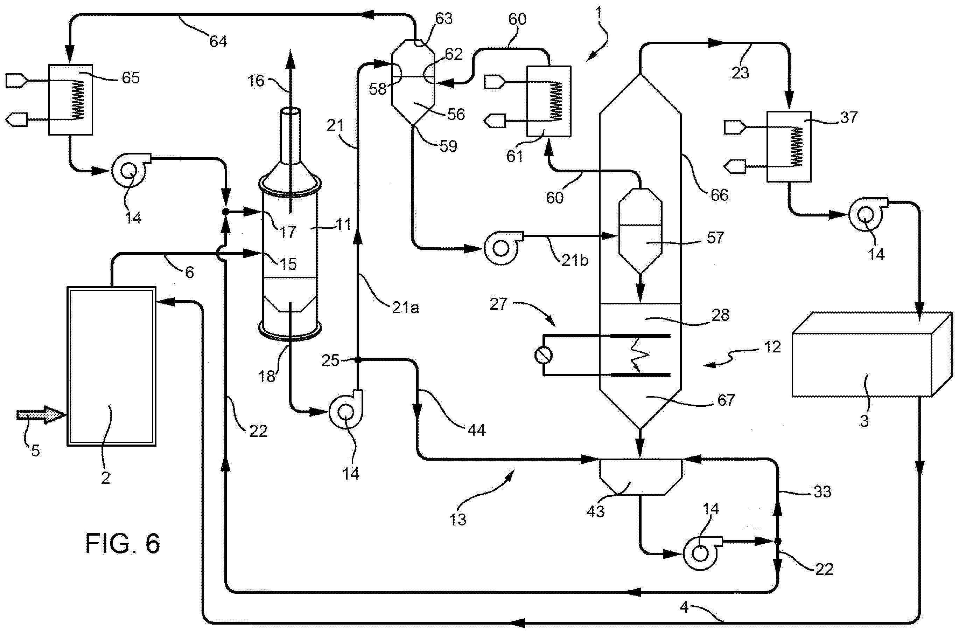

In the embodiment in Figure 6 (particularly indicated when ammonium sulphates are used), the heat treating unit 12 still includes an oven 28, for example an electric oven with immersed electrodes, and a mixer 43. The recovery system 1 also includes a crystallizer 56 (or other type of concentrator) located along the inlet line 21 between the scrubber 11 and the oven 28, and a hydrocyclone 57 (or other type of solid/liquid separator) positioned, for example, above the oven 28.

Before being sent to the oven 28, the recovery solution coming from the scrubber 11 is sent to the crystallizer 56 to separate the water from the recovery solution, so as to supply the oven 28 with a product, in particular one or more crystallized (or at least concentrated) ammonia salts (for example, ammonium sulphate).

The recovery solution extracted from the scrubber 11 is thus sent to the crystallizer 56 through a first section 21a of the inlet line 21 (which connects the bottom outlet 18 of the scrubber 11 to a first inlet 58 of the crystallizer 56); a second section 21b of the inlet line 21 then connects a bottom outlet 59 of the crystallizer 56 to the hydrocyclone 57.

Heat is provided inside the crystallizer 56 to deposit slurry containing an aqueous solution and solids on the bottom of an internal crystallization chamber of the crystallizer 56; this slurry is sent, via section 21b, to the hydrocyclone 57, where the solids are separated from the solution. The solids (with traces of water) are sent to the oven 28; the solution is sent through a line 60 to a reboiler 61 (or other heat exchanger) and subsequently to a second inlet 62 of the crystallizer 56, to provide heat for the crystallization.

A vapour phase taken from the crystallizer 56, specifically from a top outlet 63 of the crystallizer 56, runs through a vapour line 64 and, after optionally passing through a heat exchanger 65, is fed back into the scrubber 11, for example, by flowing into the return line 22.

To avoid handling the solids, the hydrocyclone 57 is advantageously located on top of the oven 28, or inside the oven 28 or a shell 66 that also contains the oven 28, over a bottom zone 67 of the oven 28 occupied by the molten bath that forms in the oven 28: in this way, the solids separated in the hydrocyclone 57 are transferred by falling into the oven 28 where fusion of the salt takes place. In addition, it is also possible to recover part of the heat of the ammonia vapours produced in the oven 28 to keep the hydrocyclone 57, and the solution leaving it, hot.

Optionally, instead of being sent to the crystallizer 56, part of the recovery solution is sent to the mixer 43 through the auxiliary line 44, which runs from the inlet line 21 upstream of the crystallizer 56 (in particular, from branch point 25).

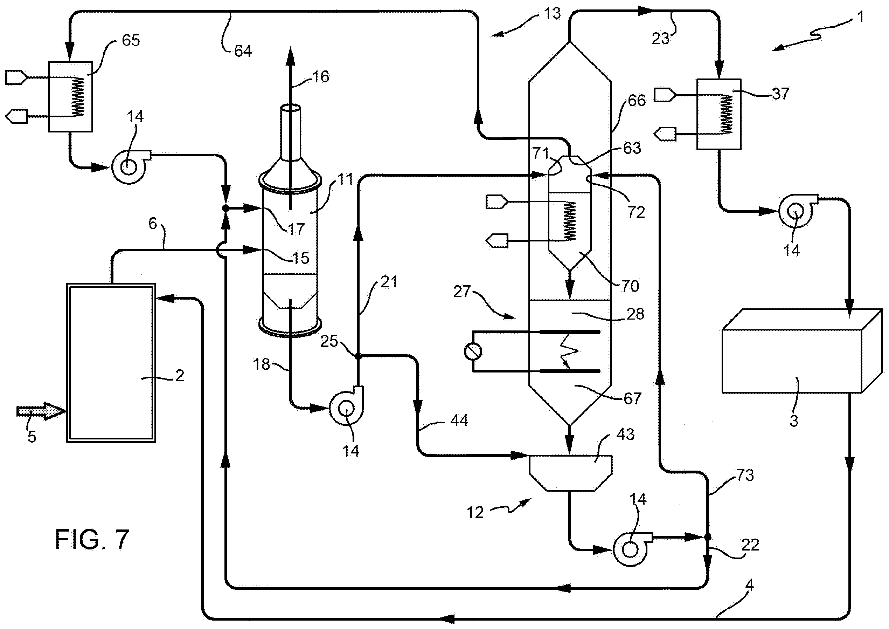

Similarly, in the embodiment in Figure 7 (also particularly indicated when ammonium sulphates are used) the recovery solution coming from the scrubber 11 is concentrated, via the removal of water, before being sent to the oven 28.

In this case, the recovery system 1 comprises an evaporator 70 (concentrator), which receives the recovery solution and separates water from the recovery solution, so as to supply the oven 28 with a concentrated product, in particular one or more ammonia salts (for example, ammonium sulphate).

A portion of the recovery solution extracted by the scrubber 11 is sent to the evaporator 70 through the inlet line 21 (which connects the bottom outlet 18 of the scrubber 11 to a first inlet 71 of the evaporator 70).

The remainder of the recovery solution is instead sent to the mixer 43 through the auxiliary line 44, which runs from the inlet line 21 upstream of the evaporator 70 (in particular, from branch point 25).

The evaporator 70 is heated, for example via low-pressure steam, to evaporate water and form the concentrate (containing ammonium sulphate in particular) that is discharged into the oven 28.

Advantageously, the evaporator 70 is located on top of the oven 28, or inside the oven 28 or the shell 66 that also contains the oven 28, over the bottom zone 67 of the oven 28 occupied by the molten bath that forms in the oven 28: in this way, the concentrate formed in the evaporator 70 may be discharged directly into the oven 28 and it is possible to recover part of the heat of the ammonia vapours produced in the oven 28 to heat the evaporator 70.

Advantageously, the evaporator 70 has a second inlet 72 connected to a service line 73 that runs from the return line 22 to transfer a portion of the reactant composition circulating in the return line 22 (solution formed in the mixer 43 and containing the molten mixture produced in the oven 28 and mixed with a portion of the recovery solution) to the evaporator 70.

In this way, ammonium bisulphate is added in the evaporator 70 and it is possible to eliminate a larger amount of water, obtaining a molten salt of ammonium bisulphate containing ammonium sulphate; in fact, ammonium bisulphate has a melting point of 147°C, so that if ammonium bisulphate is added to the solution of ammonium sulphate, in the evaporator 70 the water is eliminated and the ammonium sulphate dissolved in the molten ammonium bisulphate remains on the bottom, at a temperature close to or higher than 147°C.

A vapour phase taken from the evaporator 70, specifically from a top outlet 63 of the evaporator 70, runs through a vapour line 64 and, after optionally passing through a heat exchanger 65, is fed back into the scrubber 11, for example, by flowing into the return line 22.

In all of the above described embodiments, the gaseous ammonia produced in the heat treating unit 12 is condensed in the condensation unit 37 and then sent to the urea plant 3, for reuse.

Finally, it is understood that further modifications and variants may be made to the method described and illustrated herein without departing from the scope of the appended claims.

## Claims

1. A recovery method for processing a gas stream from a solidification unit (2) of a urea plant (3), in particular a cooling air stream used in the solidification unit (2) and containing ammonia; comprising the steps of:
- processing the gas stream from the solidification unit (2) in a scrubber (11) to remove ammonia from said gas stream by reaction with at least one reactant, in particular an acid or acid salt capable of capturing the ammonium group and forming an ammonia salt;
- drawing a recovery solution, containing at least one ammonia salt, from the scrubber (11);
- heat treating said recovery solution by heating it to a controlled temperature such as to degrade said at least one ammonia salt and produce gaseous ammonia and at least one further acid ammonia salt or corresponding acid capable of capturing the ammonium group; and forming a molten or liquid reactant composition containing said at least one acid ammonia salt or corresponding acid;
- feeding at least part of the reactant composition to the scrubber (11), to remove ammonia from the gas stream from the solidification unit (2); and
- recovering the ammonia produced in the heat treating step and feeding it to the urea plant (3);
wherein the scrubber (11) is fed with the reactant composition produced in the heat treating step, with the addition of a recycled portion of the recovery solution and without adding fresh reactants,
and wherein the recovery solution produced in the scrubber (11) is an aqueous solution of ammonium sulphate; and the reactant composition contains ammonium bisulphate produced in the heat treating step, which is performed within a temperature range of 300-320°C so as to cause degradation of the ammonia compounds contained in the recovery solution to give gaseous ammonia, but to avoid degradation of the ammonia salts or the corresponding acids to give gaseous compounds that would pollute the recovered ammonia.

2. A recovery method according to claim 1, comprising the step of condensing the ammonia produced in the heat treating step, before feeding it to the urea plant (3).

3. A recovery method according to one of claims 1 or 2, wherein the recovery solution is an aqueous solution of ammonium sulphate and the heat treating step is performed at a temperature of around 312°C.

4. A recovery method according to one of claims 1 to 3, comprising the step of returning a recycle portion of the recovery solution to the scrubber (11), without subjecting the recycle part to the heat treating step.

5. A recovery method according to one of claims 1 to 4, comprising the step of mixing the reaction composition, produced in the heat treating step, with part of the recovery solution from the scrubber (11) and not subjected to the heat treating step.

6. A recovery method according to one of claims 1 to 5, comprising the step of recirculating a part of the reactant composition to the heat treating step.

7. A recovery method according to one of claims 1 to 6, comprising a distillation step, in which a gaseous phase, containing water and ammonia and produced in the heat treating step, is distilled to separate the water from the ammonia.

8. A recovery method according to one of claims 1 to 7, comprising a crystallization step to concentrate the recovery solution from the scrubber (11) prior to the heat treating step and form sludge containing an aqueous solution and solids; and a solid/liquid separation step to separate the solids, which are sent to the heat treating step, from the aqueous solution, which is fed to a reboiler (61) or other heat exchanger to supply heat to the crystallization step.

9. A recovery method according to one of claims 1 to 8, comprising a recovery solution evaporation step, to separate water from the recovery solution and supply the heat treating step with a concentrated product.

## Patentansprüche

1. Rückgewinnungsverfahren zum Verarbeiten eines Gasstroms von einer Verfestigungseinheit (2) einer Harnstoffanlage (3), insbesondere eines Kühlluftstroms, der in der Verfestigungseinheit (2) verwendet wird und Ammoniak enthält; folgende Schritte umfassend:
- Verarbeiten des Gasstroms von der Verfestigungseinheit (2) in einem Nassabscheider (11), um Ammoniak aus dem Gasstrom mittels einer Reaktion mit mindestens einem Reaktionspartner, insbesondere einer Säure oder einem Säuresalz, die bzw. das in der Lage ist, die Ammoniumgruppe aufzunehmen und ein Ammoniaksalz zu bilden, zu entfernen;
- Ziehen einer Rückgewinnungslösung, die mindestens ein Ammoniaksalz enthält, aus dem Nassabscheider (11);
- Wärmebehandeln der Rückgewinnungslösung mittels Erhitzen davon auf eine gesteuerte Temperatur, zum Beispiel zum Abbauen von dem mindestens einen Ammoniaksalz und Herstellen von Ammoniakgas und mindestens einem weiteren Säureammoniaksalz oder entsprechender Säure, das bzw. die in der Lage ist, die Ammoniumgruppe aufzunehmen; und Bilden eines geschmolzenen oder flüssigen Reaktionspartnergemischs, das das mindestens eine Säureammoniaksalz oder eine entsprechende Säure enthält;
- Fördern von mindestens einem Teil des Reaktionspartnergemischs zu dem Nassabscheider (11), um Ammoniak aus dem Gasstrom von der Verfestigungseinheit (2) zu entfernen; und
- Rückgewinnen des Ammoniaks, der in dem Wärmebehandlungsschritt hergestellt wurde, und Fördern davon zu der Harnstoffanlage (3);
wobei der Nassabscheider (11) mit dem Reaktionspartnergemisch, das in dem Wärmebehandlungsschritt hergestellt wurde, mit Hinzufügen eines recycelten Abschnitts der Rückgewinnungslösung und ohne Hinzufügen von frischen Reaktionspartnern beschickt wird,
und wobei die Rückgewinnungslösung, die in dem Nassabscheider (11) hergestellt wurde, eine wässrige Lösung von Ammoniumsulfat ist; und das Reaktionspartnergemisch Ammoniumbisulfat enthält, das in dem Wärmebehandlungsschritt hergestellt wurde, der innerhalb eines Temperaturbereichs von 300-320 °C ausgeführt wird, sodass ein Abbau der Ammoniakverbindungen, die in der Rückgewinnungslösung enthalten sind, veranlasst wird, um Ammoniakgas zu erzeugen, jedoch zu vermeiden, dass ein Abbau der Ammoniaksalze oder der entsprechenden Säuren gasförmige Verbindungen erzeugt, die den rückgewonnenen Ammoniak verunreinigen würden.

2. Rückgewinnungsverfahren nach Anspruch 1, umfassend den Schritt des Verdichtens des Ammoniaks, der in dem Wärmebehandlungsschritt hergestellt wurde, bevor er zu der Harnstoffanlage (3) gefördert wird.

3. Rückgewinnungsverfahren nach einem der Ansprüche 1 oder 2, wobei die Rückgewinnungslösung eine wässrige Lösung von Ammoniumsulfat ist und der Wärmebehandlungsschritt bei einer Temperatur von etwa 312 °C ausgeführt wird.

4. Rückgewinnungsverfahren nach einem der Ansprüche 1 bis 3, umfassend den Schritt des Rückleitens eines recycelten Abschnitts der Rückgewinnungslösung zu dem Nassabscheider (11), ohne den recycelten Abschnitt dem Wärmebehandlungsschritt zu unterziehen.

5. Rückgewinnungsverfahren nach einem der Ansprüche 1 bis 4, umfassend den Schritt des Mischens des Reaktionsgemischs, das in dem Wärmebehandlungsschritt hergestellt wird, mit einem Teil der Rückgewinnungslösung von dem Nassabscheider (11), und nicht dem Wärmebehandlungsschritt unterzogen wird.

6. Rückgewinnungsverfahren nach einem der Ansprüche 1 bis 5, umfassend den Schritt des Wiederzuführens eines Teils des Reaktionspartnergemischs zu dem Wärmebehandlungsschritt.

7. Rückgewinnungsverfahren nach einem der Ansprüche 1 bis 6, umfassend einen Destillationsschritt, in dem eine gasförmige Phase, die Wasser und Ammoniak enthält und in dem Wärmebehandlungsschritt hergestellt wird, destilliert wird, um das Wasser von dem Ammoniak zu trennen.

8. Rückgewinnungsverfahren nach einem der Ansprüche 1 bis 7, das Folgendes umfasst: einen Kristallisierungsschritt, um die Rückgewinnungslösung von dem Nassabscheider (11) vor dem Wärmebehandlungsschritt zu konzentrieren und einen Schlamm zu bilden, der eine wässrige Lösung und Feststoffe enthält; und einen Feststoff/Flüssigkeit-Trennschritt, um die Feststoffe, die zu dem Wärmebehandlungsschritt geschickt werden, von der wässrigen Lösung, die zu einem Wiederverdampfer (61) oder anderen Wärmetauscher gefördert wird, um Wärme für den Kristallisierungsschritt zu liefern, zu trennen.

9. Rückgewinnungsverfahren nach einem der Ansprüche 1 bis 8, umfassend einen Rückgewinnungslösung-Verdampfungsschritt, um Wasser von der Rückgewinnungslösung zu trennen und dem Wärmebehandlungsschritt ein konzentriertes Produkt zu liefern.

## Revendications

1. Procédé de récupération pour traiter un flux gazeux d'une unité de solidification (2) d'une installation de production d'urée (3), en particulier un flux d'air de refroidissement utilisé dans l'unité de solidification (2) et contenant de l'ammoniac ; comprenant les étapes de :
- traitement du flux gazeux sortant de l'unité de solidification (2) dans un épurateur (11) pour éliminer l'ammoniac dudit flux gazeux par réaction avec au moins un réactif, en particulier un acide ou sel d'acide capable de capturer le groupe ammonium et de former un sel ammoniac ;
- extraction d'une solution de récupération, contenant au moins un sel d'ammoniac, de l'épurateur (11) ;
- traitement thermique de ladite solution de récupération par chauffage jusqu'à une température contrôlée de façon à dégrader ledit au moins sel ammoniac et à produire de l'ammoniac gazeux et au moins un autre sel ammoniac acide ou acide correspondant capable de capturer le groupe ammonium ; et formation d'une composition de réactif à l'état fondu ou liquide contenant ledit au moins sel ammoniac acide ou acide correspondant ;
- introduction d'au moins une partie de la composition de réactif dans l'épurateur (11), pour éliminer l'ammoniac du flux gazeux sortant de l'unité de solidification (2) ; et
- récupération de l'ammoniac produit dans l'étape de traitement thermique et introduction de celui-ci dans l'installation de production d'urée (3) ;
dans lequel l'épurateur (11) est alimenté par la composition de réactif produite dans l'étape de traitement thermique, avec ajout d'une partie recyclée de la solution de récupération et sans ajout de réactifs frais,
et dans lequel la solution de récupération produite dans l'épurateur (11) est une solution aqueuse de sulfate d'ammonium ; et la composition de réactif contient le bisulfate d'ammonium produit dans l'étape de traitement thermique, qui est mise en oeuvre dans une plage de températures de 300-320 °C de façon à provoquer la dégradation des composés ammoniac contenus dans la solution de récupération et à générer de l'ammoniac gazeux, mais à éviter la dégradation des sels ammoniac ou acides correspondants qui pollueraient l'ammoniac récupéré.

2. Procédé de récupération selon la revendication 1, comprenant l'étape de condensation de l'ammoniac produit dans l'étape de traitement thermique, avant son introduction dans l'installation de production d'urée (3).

3. Procédé de récupération selon l'une des revendications 1 ou 2, dans lequel la solution de récupération est une solution aqueuse de sulfate d'ammonium et l'étape de traitement thermique est mise en oeuvre à une température d'environ 312 °C.

4. Procédé de récupération selon l'une des revendications 1 à 3, comprenant l'étape de retour d'une partie recyclée de la solution de récupération vers l'épurateur (11), sans soumission de la partie recyclée à l'étape de traitement thermique.

5. Procédé de récupération selon l'une des revendications 1 à 4, comprenant l'étape de mélange de la composition de réactif, produite dans l'étape de traitement thermique, avec la partie de la solution de récupération sortant de l'épurateur (11) et non soumise à l'étape de traitement thermique.

6. Procédé de récupération selon l'une des revendications 1 à 5, comprenant l'étape de remise en circulation d'une partie de la composition de réactif vers l'étape de traitement thermique.

7. Procédé de récupération selon l'une des revendications 1 à 6, comprenant une étape de distillation, dans laquelle une phase gazeuse, contenant de l'eau et de l'ammoniac et produite dans l'étape de traitement thermique, est distillée pour séparer l'eau de l'ammoniac.

8. Procédé de récupération selon l'une des revendications 1 à 7, comprenant une étape de cristallisation pour concentrer la solution de récupération sortant de l'épurateur (11) avant l'étape de traitement thermique et former une suspension épaisse contenant une solution aqueuse et des fractions solides ; et une étape de séparation solides/liquides pour séparer les fractions solides, qui sont envoyées vers l'étape de traitement thermique, de la solution aqueuse, qui est introduite dans un rebouilleur (61) ou autre échangeur de chaleur pour fournir de la chaleur à l'étape de cristallisation.

9. Procédé de récupération selon l'une des revendications 1 à 8, comprenant une étape d'évaporation de la solution de récupération, pour séparer de l'eau de la solution de récupération et alimenter l'étape de traitement thermique en produit concentré.
